# EUROPÄISCHE PATENTSCHRIFT

(11) **EP 2 016 416 B1**
(45) Veröffentlichungstag und Bekanntmachung des Hinweises auf die Patenterteilung: **19.12.2012**
(21) Anmeldenummer: 07724987.8
(22) Anmeldetag: 08.05.2007
(51) Int. Cl.: G01N 33/574, G01N 33/576, G01N 33/68

(54) **IN VITRO VERFAHREN ZUR ERKENNUNG UND FRÜHERKENNUNG UND ZUR BEGLEITENDEN KONTROLLE DER THERAPIE VON ARZNEI- UND SUCHTMITTELINDUZIERTEN LEBERSCHÄDEN**
IN VITRO METHOD FOR THE IDENTIFICATION AND EARLY IDENTIFICATION AND FOR THE CONCOMITANT MONITORING OF THE THERAPY OF DRUG- AND ADDICTIVE SUBSTANCE-INDUCED LIVER DAMAGE
PROCEDE IN VITRO D'IDENTIFICATION ET DE DIAGNOSTIC PRECOCE ET CONTROLE ASSOCIE DE LA THERAPIE DES DOMMAGES HEPATIQUES INDUITS PAR MEDICAMENTS OU SUBSTANCES ADDICTIVES

(30) Priorität: 08.05.2006 DE 102006021406
(43) Veröffentlichungstag der Anmeldung: 21.01.2009
(73) Patentinhaber: B.R.A.H.M.S GmbH, 16761 Hennigsdorf (DE)
(72) Erfinder: BERGMANN, Andreas, 13465 Berlin (DE); STRUCK, Joachim, 13465 Berlin (DE); MORGENTHALER, Nils, G., 13503 Berlin (DE)
(74) Vertreter: Andrae, Steffen
(86) Internationale Anmeldenummer: PCT/EP2007/004063
(87) Internationale Veröffentlichungsnummer: WO 2007/128570

(56) Entgegenhaltungen:
- EP-A- 1 355 159
- WO-A-00/73322
- US-A1- 2004 235 953
- NAVARRO VICTOR J ET AL: "Current concepts - Drug-related hepatotoxicity" NEW ENGLAND JOURNAL OF MEDICINE, Bd. 354, Nr. 7, Februar 2006 (2006-02), Seiten 731-739, XP002455214 ISSN: 0028-4793
- OZAKI M ET AL: "ENZYME-LINKED IMMUNOSORBENT ASSAY OF CARBAMOYLPHOSPHATE SYNTHETASE I: PLASMA ENZYME IN RAT EXPERIMENTAL HEPATITIS AND ITS CLEARANCE" ENZYME PROTEIN, KARGER, BASEL, CH, Bd. 48, Nr. 4, 1994, Seiten 213-221, XP008018977 ISSN: 1019-6773

### SEQUENCE LISTING

<110> B.R.A.H.M.S Aktiengesellschaft
<120> *In vitro* Verfahren zur Erkennung und Früherkennung und zur begleitenden Kontrolle der Therapie von arzneimittelinduzierten Leberschäden
<130> 4927PCT
<140>
   <141>
<160> 2
<170> PatentIn version 3.2
<210> 1
   <211> 17
   <212> PRT
   <213> Artificial
<220>
   <223> Synthetic
<400> 1
   Cys Glu Phe Glu Gly Gln Pro Val Asp Phe Val Asp Pro Asn Lys Gln
   1               5                   10                  15
   Asn
<210> 2
   <211> 15
   <212> PRT
   <213> Artificial
<220>
   <223> Synthetic
<400> 2
   Cys Phe His Gly Thr Ser Ser Arg Ile Gly Ser Ser Met Lys Ser
   1               5                   10                  15

## Beschreibung

Die Erfindung betrifft ein neues *in vitro* Verfahren zur Früherkennung von arzneimittelinduzierten oder suchtmittelinduzierten Leberschäden und zur Erkennung der bereits erreichten Stufe der Leberschädigung durch Bestimmung eines für diesen Zwecke noch nicht verwendeten oder vorgeschlagenen Biomarkers in der Zirkulation eines humanen Patienten im Rahmen einer Multiparameterbestimmung.

Es ist bekannt, dass zahlreiche an sich nützliche und in großem Umfange verschriebene Arzneimittel bei einzelnen dafür disponierten Personen oder unter bestimmten ungünstigen Voraussetzungen (z.B. zusätzlichen Belastungen des Organismus durch weitere Arzneimittel, Alkoholkonsum, andere externe Belastungen, Ernährungszustand und -gewohnheiten) zu Leberschäden führen können.

Derartige, im Rahmen der vorliegenden Anmeldung auch als "potentiell leberschädigende Arzneimittel" bezeichnete Arzneimittel sind in den verschiedensten Wirkstoffklassen mit Anwendungen für nahezu alle klinischen Indikationen zu finden, wobei es für einige Arzneimittel bekannt ist, dass bei ihnen das Risiko besonders hoch ist.

Die von den Arzneimitteln bewirkten Leberschäden äußern sich klinisch in vielfältigen Symptomen, die als solche nicht besonders aussagekräftig sind. Zu nennen sind z.B. Appetitlosigkeit, Abgeschlagenheit, Schwindel, Gewichtsverlust, Übelkeit, Erbrechen, Fieber, Schmerzen im rechten Oberbauch, Arthralgien, Myalgien, Juckreiz, Ausschläge und Verfärbung der Ausscheidungen. Das auffälligste Symptom, das allerdings erst in einem relativ weit fortschrittenen Zustand auftritt, ist ein Gelbwerden der Augen oder sogar der Haut, was dann Anlass für eine sofortige gastroenterologische Diagnostik sein muss, da schwere und möglicherweise irreversible Schädigungen der Leber zu befürchten sind. Eine Diskussion der Problematik findet sich z.B. in R. Teschke, Dt. Ärzteblatt 2001, 98:A2584-2589; oder in Victor J. Navarro, M.D., und John R. Senior, Drug-Related Hepatotoxixity, N Engl J Med 2006; 354:731-739.

Da mögliche Leberschäden aufgrund von Arzneimittelnebenwirkungen im Interesse der Patienten, der Volksgesundheit und der Vermeidung negativer volkswirtschaftlicher Folgekosten möglichst früh erkannt werden sollten, die bisher zur Verfügung stehenden diagnostischen Mittel aber nicht oder nur unzureichend für eine solche Früherkennung geeignet sind, besteht ein erheblicher Bedarf nach neuen zuverlässigen und gut bestimmbaren diagnostischen Parametern für die Früherkennung von Leberschäden, die auf die externe Zufuhr von leberschädigen Substanzen wie insbesondere von Arzneimitteln, jedoch auch von schädlichen Genuss- und Suchtmitteln (Rauschgiften, Aufputschmitteln, Alkohol) oder auch anderen Substanzen zurückzuführen sind, gegen die Personen und Personengruppen exponiert sind.

Da Blutuntersuchungen zur ärztlichen Routine gehören, wäre insbesondere ein neuer zuverlässiger und einfach und sicher in einer Blutprobe zu bestimmender Biomarker, der die o.g. Anforderungen erfüllt, höchst erwünscht.

Die vorliegende Erfindung betrifft die Identifizierung eines neuen derartigen Biomarkers, und seine Verwendung im Rahmen einer Multiparameterbestimmung zur Früherkennung von Leberschäden aufgrund des Gebrauchs von Arzneimitteln oder Suchtmitteln und zur Erkennung der Stufe der erreichten Leberschädigungen, wie es durch Anspruch 1 beschrieben wird.

Bevorzugte bzw. hervorgehobene Ausführungsformen finden sich in den Unteransprüchen 2 bis 8.

Die Erfindung betrifft *in vitro*-Diagnoseverfahren, bei denen man den bereits in einem Frühstadium von arzneimittelbedingten oder auch suchtmittelbedingten Leberschädigungen im Blut erscheinenden bzw. mit erhöhten Konzentrationen messbaren humoralen Biomarker CPS 1 bzw. dessen Fragmente zur Erkennung von Leberschäden verwendet, die durch den Gebrauch von Arzneimitteln (Pharmazeutika, Pharmaka) oder Suchtmitteln (Rauschgift, Alkohol) verursacht sind.

Die Begriffe "Arzneimittel" bzw. "Suchtmittel" werden in der vorliegenden Anmeldung in einem umfassenden, weiten Sinne für physiologisch aktive Substanzen gebraucht, die eine Person zu sich nimmt oder verabreicht bekommt und die unter bestimmten Bedingungen und/oder bei bestimmten entsprechend disponierten Personen eine Schädigung der Leber hervorrufen können. Damit umfasst der Begriff "Arzneimittel", ohne dass die folgende bespielhafte Aufzählung erschöpfend sein soll, insbesondere alle verschreibungspflichtigen oder frei verkäuflichen (OTC) Stoffe und Zubereitungen von Stoffen im Sinne des Arzneimittelgesetzes, die dazu bestimmt sind, durch Anwendung am oder im menschlichen Körper Krankheiten, Leiden, Körperschäden oder krankhafte Beschwerden zu heilen, zu lindern, zu verhüten oder zu erkennen. Die Arzneimittel können von der pharmazeutischen Industrie synthetisierte und/oder zu Arzneimitteln verarbeitete Wirkstoffe und Wirkstoffzubereitungen sein, sie können aber auch in der Naturheilkunde verwendete Stoffe und Zubereitungen von Stoffen sein, z.B. Phytopharmaka oder sog. funktionelle Nahrungsergänzungsmittel.

Ferner soll der Bereich der vorliegenden Erfindung nicht auf "Arzneimittel" im obigen breit definierten Sinne beschränkt sein, sondern auch die Wirkungen anderer pharmakologisch wirksamer Substanzen erfassen, die Menschen zu anderen als zu Heilzwecken zu sich nehmen, wobei solche andere Substanzen hierin unter dem Begriff "Suchtmittel" zusammengefasst werden und z.B. Rauschgifte und potentiell gesundheitsschädliche Genussmittel umfassen.

Bei einer noch grundsätzlicheren Betrachtungsweise betrifft das erfindungsgemäße Verfahren einen - allerdings für die medizinische Praxis und die Volksgesundheit besonders wichtigen - Spezialfall der Früherkennung von bestimmten Leberschäden, die durch nahezu beliebige leberschädigende (lebertoxische) Substanzen hervorgerufen werden. Somit lässt sich der Bereich der Erfindung auch auf die gesundheitsschädlichen Wirkungen von pharmakologisch wirksamen Substanzen erstreken, die die Personen nicht bewusst zu sich nehmen, sondern die sie z.B. als Verunreinigungen von Nahrungsmitteln oder als toxische Umweltchemikalien aufnehmen. Das Verfahren ist z.B. auch zur Früherkennung von Leberschäden geeignet, die durch die Einwirkung von möglicherweise unbekannten Substanzen ausgelöst werden, denen Patienten, einzelne Individuen oder bestimmte Bevölkerungs- oder Berufsgruppen ausgesetzt sind. So kann das Verfahren z.B. dazu dienen, in Bevökerungsgruppen mit bestimmten Lebensweisen oder in bestimmten Berufsgruppen lebertoxische Substanzen aufzuspüren, wenn sich bei einer solchen Bevölkerungsgruppe in statistisch signifikanter Weise Erhöhungen der CPS 1-Konzentrationen im Blut feststellen lassen.

Beispielhaft und ohne dass dadurch eine Beschränkung des Schutzbereichs der Erfindung herbeigeführt werden soll, lassen sich als Arzneimittelgruppen, denen die potentiell leberschädigenden Arzneimittel zugeordnet werden können, nennen nicht-steroidale antiinflammatorische Arzneimittel (NSAIDs), andere Schmerzmittel, anabole Steroide, hormonale Kontrazeptiva, Gichtmittel, Statine, Inhalationsnarkotika, Antibiotika, Sulfonamide, Tuberkulostatika, Antitumormitteln, Psychopharmaka, Herz-Kreislauf-Mittel, insbesondere blutdrucksenkende Mittel, Phytopharmaka.

Als speziellere deartige Arzneimittel bzw. Arzneimittelgruppen lassen sich beispielhaft nennen Paracetamol, Tetracycline, Ibuprofen, Chlorpromazin, Captopril, Enalapril, Halothan, Kavakava, Streptomycin, Isoniazid und/oder Rifampicin.

Nicht in den eingentlichen Bereich der vorliegenden Erfindung fallen Leberschäden, die durch infektiöse Partikel mikrobiellen oder viralen Ursprungs, die nicht als "Arzneimittel" oder "Suchtmittel" zu bezeichnen sind, verursacht werden.

Die vorliegende Erfindung beruht auf der überraschenden Feststellung, dass bei arzneimittelbedingten sowie den verwandten suchtmittelbedingten Leberschädigungen bereits in einem Frühstadium, in dem eine Leberschädigungen noch nicht klinisch manifest wird, deutlich erhöhte Konzentrationen des Enzyms Carbamoylphosphat Synth(et)ase (CPS 1) bzw. eine starke CPS 1-Immunreaktivität nachweisbar sind, und zwar in einem klaren Unterschied zu Personen, die zwar mit den gleichen Arzneimitteln behandelt werden, bei denen sich jedoch - z.B. weil sie als Individuen weniger empfindlich reagieren oder bestimmten weiteren zusätzlichen Risikofaktoren nicht ausgesetzt sind - keine Leberschädigungen entwikeln. Dieser Befund macht CPS 1 zu einem humoralen Biomarker, der insbesondere zur Früherkennung arzneimittelbedingter Leberschädigungen geeignet ist.

Unter Verwendung eines Immunoassays, der von der Anmelderin im Zusammenhang mit der Sepsisdiagnose entwickelt worden war und der selektiv den Nachweis bzw. die Konzentrationsmessung von CPS 1 in einem Serum oder Plasma eines humanen Patienten ermöglicht, wurde im Hause der Anmelderin nachgewiesen, dass durch eine CPS 1-Bestimmung arzneimittelbedingte Leberschädigungen besonders frühzeitig erkannt werden können, und zwar bevor klinische Symptome einer Leberschädigung auftreten und bevor andere bekannte Marker (Leberenzyme), die als klinische Marker für Lebererkrankungen eingeführt sind, signifikant erhöht gefunden werden, und bevor die Leberschädigungen möglicherweise irreversibel werden.

Für die medizinische Diagnostik spielten CPS 1 bzw. CPS 1-Fragmente mit CPS 1-Immunreaktivität bisher noch keine praktische Rolle.

Das Enzym CPS 1 ((E.C. 6.3.4.16) selbst ist jedoch seit langem gut bekannt. Es katalysiert die Umwandlung von Ammoniak, Bicarbonat und 2 ATP unter Bildung von Carbamoylphosphat im ersten Schritt des-Harnstoffzyklus. Es spielt dabei auch eine Rolle bei der Biosynthese von Arginin, das seinerseits ein Substrat für die Biosynthese von NO, z.B. bei einem Endotoxin-Schock, ist (vgl. Shoko Tabuchi et al., Regulation of Genes for Inducible Nitric Oxide Synthase and Urea Cycle Enzymes in Rat Liver in Endotoxin Shock, Biochemical and Biophysical Research Communications 268, 221-224 (2000)). CPS 1 ist von dem cytosolischen Enzym CPS 2 (E.C. 6.3.5.5.) zu unterscheiden, das ebenfalls eine Rolle im Harnstoffzyklus spielt, jedoch das Substrat Glutamin verarbeitet. Es ist bekannt, dass CPS 1 in Mitochondrien lokalisiert ist und im Lebergewebe in dieser Form in großen Mengen (es macht von 2-6% des Leber-Gesamtproteins aus) vorkommt. Seine Aminosäuresequenz und genetische Lokalisierung ist seit langem bekannt (vgl. Haraguchi Y. et al, Cloning and sequence of a cDNA encoding human carbamyl phosphate synthetase I: molecular analysis of hyperammonemia, Gene 1991, Nov. 1; 107 (2):335-340; vgl. auch die Veröffentlichung WO 03/089933 A1 der Anmelderin). Zu seiner physiologischen Rolle kann verwiesen werden auf Reviewartikel wie z.B. H.M.Holden et al., Carbamoyl phosphate synthetase: an amazing biochemical odyssey from substrate to product, CMLS, Cell.Mol.Life Sci. 56 (1999) 507-522 und die darin referierte Literatur, sowie die Einleitung der Veröffentlichung Mikiko Ozaki et al., Enzyme-Linked Immunosorbent Assay of Carbamoylphosphate Synthetase I: Plasma Enzyme in Rat Experimental Hepatitis and Its Clearance, Enzyme Protein 1994, 95:48:213-221.

Gemäß Li Yin et al., Participation of different cell types in the restitutive response of the rat liver to periportal injury induced by allyl alcohol, Journal of Hepatology 1999, 31:497-507, läßt sich bei einer Leberschädigung durch Allylakohol bei histologischen Untersuchungen nach drei Tagen in allen Hepatocyten eine Steigerung der CPS 1-Expression beobachten.

Es wurde ferner festgestellt, dass sich im Rattenmodell bei einer durch Verabreichung von Galactosamin experimentell erzeugten akuten Hepatitis im Rattenplasma eine stark erhöhte immunologische CPS 1-Aktivität findet (nachgewiesen mit einem ELISA mit anti-Ratten CPS 1 IgG aus Kaninchen), und zwar 24-48 h nach der Behandlung mit dem Hepatitis-auslösenden Galactosamin. Im Rattenplasma wurden während der akuten Hepatitis auch zunehmend CPS 1-Fragmente mit molaren Massen von ca. 140 und 125 kDa ohne sonstige nähere Charakterisierung (Sequenzzuordnung) erkennbar, während bei einer begleitenden Immunoblotting-Analyse in humanen Autopsie-Proben keine CPS 1-Fragmente mit CPS 1-Immunreaktivität beobachtet werden konnten (Mikiko Ozaki et al., loc. cit.).

In einer Arbeit von Liu Tong-hua et al., Carbamoyl Phosphate Synthetase 1, A Novel Marker for Gastric Carcinoma, Chinese Medical Journal, 102(8):630-638, 1989 werden Ergebnisse von immunocytometrischen Untersuchungen an Gewebsproben aus verschiedenen operative entfernten Tumoren auf eine mögliche Anwesenheit von CPS 1 berichtet. Die Autoren fanden nur in Karzinomgewebe aus dem Magen Hinweise auf CPS 1-Immunreaktivität, dagegen nicht in anderen Tumorgeweben (Ösophagus, Dickdarm, Pankreas, Lunge, Brust, Ovarium, Niere, Prostata und Harnblase). Sie leiten daraus eine mögliche Eignung von CPS 1 als selektiver Gewebemarker, d.h. zellulärer Tumormarker, für Magenkrebs ab. Ein mögliches Auftreten von CPS 1 in der Zirkulation wird nicht diskutiert.

Bisher wurde die Bestimmung von humaner CPS 1 in humanem Serum bzw. Plasma, und zwar in Veröffentlichungen der Anmelderin, nur bei Sepsispatienten (vgl. WO 03/089933 A1; s. auch: Joachim Struck et al., Release of the Mitochondrial Enzyme Carbamoyl Phosphate Synthase under Septic Conditions, in: Shock, Vol. 23, No.6, S.533-538, 2005) und Tumorpatienten (DE 10 2004 045 705 A1) beschrieben. Sepsispatienten stellen eine von Patienten, die wegen verschiedenen Erkrankungen oder gesundheitlichen Auffälligkeiten über längere Zeiträume mit Arzneimitteln behandelt werden, klar unterschiedene Patientenpopulation dar. Das Gleiche gilt für Personen, die über über ausgedehnte Zeiträume potentiell schädliche Sucht- oder Genussmittel (Rauschgifte, Alkohol) zu sich nehmen oder besonderen andersartigen Risiken ausgesetzt sind.

Bei Sepsispatienten wird eine hochakute potentiell lebensbedrohende Erkrankung typischerweise in der Intensivstation eines Krankenhauses stationär überwacht und behandelt, während eine übliche Arzneimittelbehandlung, z.B. eine Behandlung mit Schmerzmitteln, blutdrucksenkenden Mitteln oder Psychopharmaka, häufig nur der Linderung oder Beseitigung von bestimmten Krankheitssymptomen bei Personen dient, die sich ohne diese Symptome weitgehend gesund fühlen.

Bei der Bestimmung von humaner CPS 1 in Patientenseren und - plasmen gemäß der vorliegenden Erfindung kann grundsätzlich so vorgegangen werden, wie in der Veröffentlichung WO 03/089933 A1 der Anmelderin im Zusammenhang mit der Bestimmung von CPS 1 als Sepsismarker beschrieben wurde. Das im Experimentellen Teil der vorliegenden Anmeldung beschriebene immundiagnostische Bestimmmungsverfahren ist dem Verfahren, das bereits in der o.g. Anmeldung WO 03/089933 A1 der Anmelderin beschrieben wurde, nahe verwandt.

Es ist.zusätzlich ausdrücklich darauf hinzuweisen, dass es bei der erfindungsgemäßen Bestimmung von CPS 1 je nach Assaydesign zu einer gleichzeitigen Bestimmung von CPS 1 sowohl in Form des im wesentlichen vollständigen Moleküls als auch in Form von in der biologischen Flüssigkeit möglicherweise vorhandenen anderen, kürzeren Bruchstücken (physiologisch vorkommenden Teilpeptiden) der vollständigen CPS 1 kommen kann. Wenn in der vorliegenden Anmeldung von einer "Bestimmung von CPS 1" gesprochen wird, soll auch eine Bestimmung, bei der neben oder anstelle des vollständigen Enzyms CPS 1 dessen immunologisch mitreagierenden Fragmente bestimmt oder mitbestimmt werden, gemeint sein. Jede unsachgemäße einengende Auslegung der Lehre der vorliegenden Erfindung, insbesondere was die speziellere Natur des bestimmten Analyten CPS 1 angeht, soll ausgeschlossen sein. Auch eine Kombinationsmessung, bei der gleichzeitig andere Leberenzyme bestimmt werden, liegt ausdrücklich im Rahmen der vorliegenden Erfindung.

In Plasma bzw. Serum nachweisbare CPS 1 eignet sich im Lichte der nachfolgend gezeigten Ergebnisse der Vermessung eines ersten Patientenkollektivs hervorragend zum Nachweis, und dabei insbesondere zu einem frühen Nachweis vor ihrer klinischen Manifestation, und zur Verlaufs- und Therapiekontrolle von arzneimittelbedingten Leberschäden.

Für die eigentliche CPS 1-Bestimmung sind Immunoassays irgendeines geeigneten Assaydesigns bevorzugt.

Die immundiagnostischen Verfahren zur Bestimmung von CPS 1 in einer biologischen Probe können auf beliebigen bekannten Verfahrensprinzipien der Immundiagnostik beruhen, die zum Nachweis und zur Messung von Antigenen angewandt werden. Vorzugsweise wird CPS 1 mit Hilfe eines Ligandenbindungsassays bestimmt, bei dem man zur Bindung und Markierung geeignete spezifische Antikörper in immobilisierter Form bzw. markierter bzw. markierbarer Form verwendet.

Auch kompetitive Assayformate können besondere Vorteile bieten. Vorzugsweise wird dabei nicht mit einer Enyzmmarkierung gearbeitet, sondern es wird eine andere Markierung gewählt, z.B. eine Markierung für eine Chemilumineszenz-Nachweisreaktion, z.B. ein Akridiniumester. Selbstverständlich ist vorzugsweise ein solcher Assay zur CPS 1-Bestimmung zu verwenden, der die benötigte hohe Sensitivität im Bereich der vorkommenden CPS 1-Konzentrationen gewährleistet und eine Separierung der Messignale vom Assay-Hintergrund ermöglicht. Das Bestimmungsverfahren kann an die Chip-Technologie angepaßt sein oder als Schnelltest (Point-of-Care-Test), der z.B. immunchromatographische Autrennungs- und Nachweisschritte nutzt, ausgestaltet werden.

Bei einer bevorzugten Ausführungsform wird die immundiagnostische Bestimmung als heterogener Sandwich-Immunoassay durchgeführt, bei dem einer der Antikörper an eine beliebige Festphase, beispielsweise die Wände beschichteter Teströhrchen (z.B. aus Polystyrol; "Coated Tubes"; CT) oder an Mikrotiterplatten, zum Beispiel aus Polystyrol, oder an Partikel, beispielsweise Magnetpartikel immobilisiert ist, während der andere Antikörper einen Rest trägt, der ein direkt nachweisbares Label darstellt oder eine selektive Verknüpfung mit einem Label ermöglicht und der Detektion der gebildeten Sandwich-Strukturen dient. Auch eine zeitlich verzögerte bzw. nachträgliche Immobilisierung unter Verwendung geeigneter Festphasen ist möglich.

Grundsätzlich können alle in Assays der beschriebenen Art verwendbaren Markierungstechniken angewandt werden, zu denen Markierungen mit Radioisotopen, Enzymen, Fluoreszenz-, Chemolumineszenz- oder Biolumineszenz-Labeln und direkt optisch detektierbaren Farbmarkierungen, wie beispielsweise Goldatomen und Farbstoffteilchen, wie sie insbesondere für sog. Point-of-Care (POC) oder Schnelltests verwendet werden, gehören. Die beiden Antikörper können auch im Falle heterogener Sandwich-Immunoassays Teile eines Nachweissystems der nachfolgend im Zusammenhang mit homogenen Assays beschriebenen Art aufweisen.

Das erfindungsgemäße Verfahren kann ferner als homogenes Verfahren ausgestaltet werden, bei dem die aus den beiden Antikörpern und dem nachzuweisenden CPS 1 gebildeten Sandwichkomplexe in der flüssigen Phase suspendiert bleiben. In einem solchen Fall ist es bevorzugt, beide Antikörper mit Teilen eines Nachweissystems zu markieren, das dann, wenn beide Antikörper in einen einzigen Sandwich integriert werden, eine Signalerzeugung oder Signalauslösung ermöglicht. Derartige Techniken sind insbesondere als Fluoreszenzverstärkungs- oder Fluoreszenzlöschungs-Nachweisverfahren ausgestaltbar. Ein besonderes bevorzugtes derartiges Verfahren betrifft die Verwendung von paarweise einzusetzenden Nachweisreagenzien, wie sie beispielsweise beschrieben sind in US-A-4 822 733, EP-B1-180 492 oder EP-B1-539 477 und dem darin zitierten Stand der Technik. Sie ermöglichen eine Messung, die selektiv nur Reaktionsprodukte erfaßt, die beide Markierungskomponenten in einem einzigen Immunkomplex enthalten, direkt in der Reaktionsmischung. Als Beispiel ist auf die unter den Marken TRACE® (Time Resolved Amplified Cryptate Emission) bzw. KRYPTON® angebotene Technologie zu verweisen, die die Lehren der o.g. Anmeldungen umsetzt.

An Stelle einer immundiagnostischen Bestimmung von CPS 1 oder CPS 1-Immunreaktivität soll für diagnostische Zwecke die CPS 1-Bestimmung ggf. auch indirekt als Bestimmung einer Enzymaktivität, die der CPS 1-Aktivität oder der Restaktivität der CPS 1-Fragmente im Blut entspricht, erfolgen können. Da CPS 1 bei Gesunden in der Zirkulation nicht auftritt, kann eine messbare CPS 1 Enzymaktivität im Blut eines Patienten ein diagnostisch signifikantes Indiz für eine ernsthafte Störung der gesundheitlichen Unversehrtheit des Patienten sein.

Nachfolgend wird die Bestimmung von CPS 1 in Plasmen von Gesunden, von mit Arzneimitteln behandelten Patienten ohne Leberschädigung und von mit denselben Arzneitmitteln behandelten Patienten, bei denen es zu einer arzneimittelbedingten Leberschädigung kam, noch näher erläutert, wobei auf eine Figur und eine zugeordnete Tabelle Bezug genommen wird.

Die Figur zeigt:

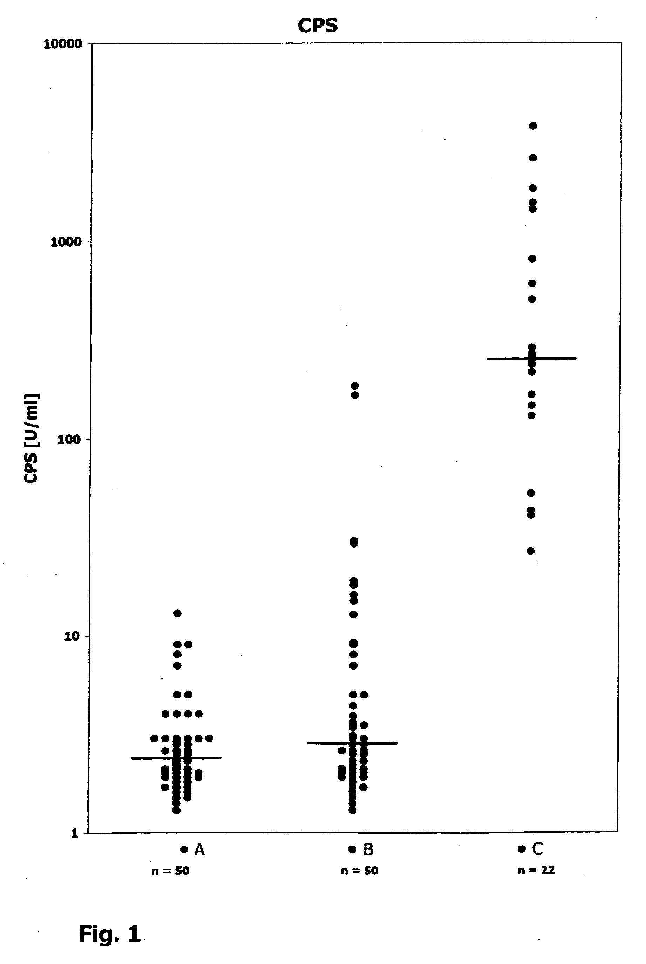

- Figur 1: die Ergebnisse der Messung der CPS 1-Konzentration (in U/ml) in Plasmen von gesunden Normalpersonen (A) und von Patienten, die mit verschiedenen Arzneimitteln behandelt wurden und bei denen es zu Leberschäden kam (C) bzw. bei den es nicht zu Leberschäden kam (B), unter Anwendung des im experimentellen Teil genauer beschriebenen Immunoassays.

### EXPERIMENTELLER TEIL

### Assaybeschreibung

### 1. Herstellung der Antikörper

### a) Immunogene

Es wurden 2 verschiedene Peptidsequenzen aus der vollständigen Sequenz der humanen CPS 1 ausgewählt, und zwar eine erste Peptidsequenz 1 (EFEGQPVDFVDPNKQN), die den Aminosäuren 184-199 der Sequenz der humanen CPS 1 entspricht (vgl. Peptid PCEN17 gemäß WO 03/089933 A1), und eine zweite Peptidsequenz (FHGTSSRIGSSMKS), die den Aminosäuren 781-794 der Sequenz des humanen CPS 1 entspricht. Jedes Peptid wurde in einer mit einem aminoterminalen Cysteinrest (Cys0) versehenen Form von der Firma Jerini (Berlin, Deutschland) synthetisiert. Die synthetisierten, für die nachfolgenden Immunisierungen verwendeten Peptide sind im Sequenzprotokoll als SEQ ID NO:1 bzw. SEQ ID NO:2 gezeigt.

### b) Antikörper

Für die Immunisierung wurden die beiden synthetisierten Peptide mit dem Hämocyanin aus *Limulus polyphemus* konjugiert, und es wurden, wie auch in der WO 03/089933 A1 beschrieben, polyklonale Antikörper in Schafen durch die Firma Ltd. Micropharm (Carmarthenshire, Großbritannien) produziert.

### 2. Reinigung der Antikörper

Die Antikörper wurden mittels ligandenspezifischer Affinitätsreinigung aufgereinigt. Hierfür wurden die Cys(0)-Peptide 1 und 2 zunächst an SulfoLink-Gel der Firma Pierce (Boston, USA) gekoppelt. Die Bindung erfolgte nach der Vorschrift des Herstellers.

Das Vorgehen war wie folgt: Polycarbonat-Säulen (-15mm x 80mm) wurden mit 5 ml Affinitätsmatrix befüllt. Nach Äquilibrierung der Säulen mit PBS (136 mM NaC1, 1,5 mM KH₂PO₄, 20,4 mM Na₂HPO₄ 2H₂O, 2,7 mM KCl, pH 7,2) wurden 5 mg der jeweiligen Peptide abgewogen, in PBS gelöst, und auf die verschlossenen Säulen gegeben. Das Gelmaterial wurde durch Schwenken homogenisiert. Nach 15minütiger Inkubation bei Raumtemperatur und Absetzen des Gelmaterials wurden die Säulen mit 5mal 3ml PBS gewaschen. Zur Absättigung freier Bindungsstellen wurden dem Säulenmaterial je 5 ml einer 50 mM L-Cysteinlösung zugesetzt, und das Gelmaterial wurde nach Homogenisierung erneut bei Raumtemperatur für 15 min inkubiert. Nach Absetzen des Gelmaterials wurde jede Säule 6mal mit 5ml einer 1M NaCl-Lösung gespült und anschließend nochmals mit PBS gespült.

Das Gelmaterial wurde mit 25ml des jeweiligen Antiserumpools gemischt und über Nacht bei Raumtemperatur unter leichtem Schwenken inkubiert. Die Serum-Gel-Gemische wurden in Polycarbonat-Säulen überführt, und überschüssiges Serum wurde entfernt. Die Säulen wurden anschließend mit 250 ml PBS gewaschen, um nicht gebundene Serumproteine zu entfernen. Die Desorption der gebundenen Antikörper erfolgte durch Elution der Säule mit 50 mM Citronensäure (pH 2,2). Das Eluat wurde in Fraktionen ä 1 ml aufgefangen. Die Proteinkonzentration jeder Fraktion wurde mit Hilfe des BCA-Proteinassay-Kits der Firma Perbio (Bonn, Deutschland) bestimmt, und die Fraktionen mit einem Proteingehalt > 1mg/ml wurden vereinigt. Die affinitätsgereinigten Antikörper wurden mittels Dialyse in PBS umgepuffert und nach erneuter Bestimmung des Proteingehalts anschließend bei 4°C gelagert.

### 3. Immobilisierung / Markierung der Antikörper

Die aufgereinigten Antikörper gegen das Peptid, das der Aminosäuresequenz 781-794 entspricht, wurden auf Polystyrolröhrchen (Startubes, 12 mm x 75 mm, Firma Greiner, Deutschland) immobilisiert. Hierfür wurden die Antikörperlösungen auf eine Proteinkonzentration von 6,7 µg/ml mit PBS verdünnt und 300 µl pro Röhrchen pipettiert (entspricht 2 µg Antikörper pro Röhrchen). Diese wurden für 20 h bei Raumtemperatur inkubiert und anschließend 3mal mit je 4 ml PBS gewaschen. Bis zur weiteren Verwendung wurden die Röhrchen bei 4°C gelagert.

Der Antikörper gegen das Peptid, das der Aminosäuresequenz 184-199 entspricht, (1 mg/ml in PBS) wurde mit Acridiniumester-N-Hydroxy-Succinimid (1 mg/ml in Acetonitril, Firma InVent, Hennigsdorf Deutschland) lumineszenzmarkiert. Für die Markierung wurden 200 µl Antikörper mit 4 µl Acridiniumester gemischt, für 20 min inkubiert, und freie Acridiniumesterbindungen wurden durch Zugabe von 40µl einer 50mM Glycin-Lösung abgesättigt. Der Markierungsansatz wurde mittels HPLC an einer BioSil 400-Gelfiltrations-Säule (Firma BioRad, München, Deutschland) von freiem Acridiniumester getrennt. Als Laufmittel wurde PBS verwendet.

### 4. Relative Kalibrierung

Um die relativen CPS 1-Konzentrationen bestimmen zu können, wurde als Standardmaterial ein Pool von Plasmen von humanen Patienten mit SIRS mit besonders hohen CPS 1-Konzentrationen verwendet. Die CPS 1-Konzentration dieses Pools wurde arbiträr auf 150 U/ml festgesetzt. Ausgehend von diesem Pool wurden durch serielle Verdünnung mit CPS 1-freiem humanem Plasma von Gesunden Standards hergestellt, denen arbiträre Konzentrationen (in relativen Einheiten, U) entsprechend ihrer Verdünnung zugeschrieben wurden.

### 5. Assay-Durchführung

50µl einer Plasma-Probe und 100µl PBS-Puffer (mit 10 mM EDTA) wurden pro Antikörper-beschichtetes Röhrchen pipettiert und 16 h bei Raumtemperatur inkubiert. Nach 3maligem Waschen mit je 1 ml PBS wurden pro Röhrchen 15 ng des markierten Antikörpers (in 200µl PBS-Puffer, 10mM EDTA) zugesetzt. Die Röhrchen wurden für weitere 2h inkubiert, und anschließend wurde ungebundener Tracer-Antikörper durch 5maliges Waschen mit je 1ml PBS entfernt. Am Röhrchen gebundener markierter Antikörper wurde mittels Lumineszenzmessung in einem Luminometer (Berthold LB 952T/16) quantifiziert.

### Messung von EDTA-Plasmen von Gesunden und von Patienten, die mit verschiedenen Arzneimitteln behandelt werden/wurden

Als Testproben für die CPS 1-Bestimmungen dienten einmal 50 Plasmen von gesunden Kontrollpersonen sowie 73 Plasmen von Patienten, die mit verschiedenen, in Tabelle 1 angegebenen Arzneimitteln behandelt wurden, für die bekannt ist, dass sie potentiell leberschädigend sein können. Die 73 Plasmen von Patienten umfassten Plasmen von 50 Patienten, bei denen kein Leberschaden festgestellt wurde, sowie 23 weitere Plasmen von Patienten, die mit den gleichen in Tabelle 1 angegebenen Arzneimitteln behandelt wurden, bei denen es jedoch zu einem Leberschaden kam.

In die o.g. Teströhrchen wurden je 50 µl Standard bzw. Probe sowie 200 µl Assaypuffer pipettiert. Es wurde 18 Stunden bei 22°C unter Schütteln inkubiert. Dann wurde 4 x mit je 1 ml Waschlösung (0.1% Tween 20) pro Röhrchen gewaschen. Dann wurde in jedes Röhrchen 200 µl Assaypuffer, enthaltend 0.5 Millionen RLU des MA70-markierten Tracer-Antikörpers, pipettiert. Es wurde zwei Stunden bei 22°C unter Schütteln inkubiert. Dann wurde 4 x mit je 1 ml Waschlösung (0.1% Tween 20) pro Röhrchen gewaschen, abtropfen gelassen und die am Röhrchen gebundene Chemilumineszenz in einem Luminometer (Firma BERTHOLD, LB952T; Basisreagenzien BRAHMS AG) vermessen.

Unter Verwendung der Software MultiCalc (Spline Fit) wurde die Konzentration an CPS 1-Immunreaktivität abgelesen. Es wurden die folgenden Ergebnisse erhalten:
Der Median der bei dem Kollektiv 50 Gesunder gemessenen CPS 1-Konzentrationen lag bei 2,4 U/ml.

Die Ergebnisse für arzneimittelbehandelte Personen ohne Leberschädigung (B) und arzneimittelbehandelte Personen, bei denen es zu einer Leberschädigung kam (C), sind in der nachfolgenden Tabelle dargestellt und, neben den zusätzlichen Messwerten für die Gesunden (A), in Figur 1 graphisch dargestellt.

**Tabelle 1**

| Medikament | Behandelte Personen ohne Leberschaden (B) | Behandelte Personen mit Leberschaden (C) |
|---|---|---|
| Paracetamol | 5 | 2 |
| Statine | 5 | 4 |
| Anabole Steroide | 3 | 1 |
| Halothan | 7 | 2 |
| Chlorpromazin | 5 | 3 |
| Sulfonamide | 4 | 2 |
| Tetracyline | 6 | 3 |
| Rifampicin | 3 | 3 |
| Captopril | 6 | 2 |
| Ibuprofen | 6 | 1 |

Für die Ergebnisse der Messungen bei 50 Patienten der Gruppe (B) wurde ein Median von 2,8 U/ml gefunden, für die der Gruppe (C) ein Median von 253 U/ml.

Begleitende Untersuchungen der Anmelderin haben ferner ergeben, dass es bei Leberschäden, wie sie typischerweise als Arzneimittel-Nebenwirkungen auftreten können, zu einer Schädigung der Mitochondrien und einer damit verbundenen CPS 1 Freisetzung aus den Leberzellen kommt, bevor eine Apoptose der Leberzellen bzw. eine Nekrose des Lebergewebes eintritt. Die CPS 1 Freisetzung ist somit zu einem Zeitpunkt feststellbar, zu dem noch keine anderen klinischen Symptome beobachtbar sind, die u.a. in der Folge dann auch zu Veränderungen der Konzentrationen der traditionell bestimmten Leberparameter, z.B. anderen Leberenzyme, führen, die normalerweise erst dann erhöht gemessen werden, wenn in der Leber nekrotische Prozesse eingesetzt haben.

Wird die Einwirkung der schädlichen externen Substanz, z.B. des Arzneimittels, auf der Stufe gestoppt, wenn im wesentlichen nur CPS 1 erhöht gemessen wird, können die geschädigten Mitochondrien in den ansonsten unbeschädigten Leberzellen ersetzt werden, und die Leber erholt sich. Es treten keine irreversiblen Leberschäden auf, wie sie bei einer erst später möglichen Diagnostik auf der Basis der herkömmlichen klinischen Befunde und der bekannten Leberparameter, deren Freisetzung mit nekrotischen Prozessen in Zusammenhang steht, auftreten können.

Eine prophylaktische Messung von CPS 1 bei Personen, die über längere Zeiträume mit Arzneimitteln behandelt werden, potentiell schädliche Genuss- und Suchtmittel zu sich nehmen oder aufgrund ihrer Ernährungsgewohnheiten und Lebensbedingungen einem erhöhten Risiko der Entwicklung von Leberschäden ausgesetzt sind, kann sich entwickelnde Leberschäden frühzeitig erkennbar machen, so dass Gegenmaßnahmen entsprechend frühzeitig eingeleitet werden können (z.B. die auslösenden Arzneimittel abgesetzt werden können) und Spätschäden und die damit für die Patienten und die gesamte Volkswirtschaft verbundenen Kosten vermieden werden können.

Werden neben CPS 1 die erst auf späteren Stufen der Leberschädigung im Serum messbaren herkömmlichen Leberenzyme mitbestimmt, können aus deren gleichzeitiger Nachweisbarkeit oder Nichtnachweisbarkeit direkt Schlüsse bezüglich der bereits erreichten Stufe der Leberschädigung abgeleitet werden. So bedeutet z.B. eine Nichtnachweisbarkeit einiger oder aller der parallel bestimmten weiteren Leberenzyme wie z.B. GOT (Glutamat-Oxalacetat-Transaminase), GPT (Serum-Glutamat-Pyruvat-Transaminase), GLDH (Glutamat-Dehydrogenase), γ-GT (γ-Glutamyl-Transpeptidase), Sorbitol-Dehydrogenase, alkalische Phosphatase, ALT (Alanin-Amino-Transferase), AST (Aspartat-Amino-Transferase), GDH (Glycerinphosphat-Dehydrogenase), LHD (Lactat-Dehydrogenase), Cholinesterase, LAP (Leucinamino-Peptidase), GGTP (Glutamyl-Transpeptidase) und anderer im Rahmen der Leberdiagnostik bestimmter Leberenzyme, dass der Leberschaden noch nicht weit fortgeschritten ist und z.B. durch Beendigung der Exposition rückgängig gemacht werden kann, während die gleichzeitige Nachweisbarkeit einiger oder aller der genannten Leberenzyme auf eine fortgeschrittene, evtl. bereits irreversible Leberschädiung hindeutet. Die Bestimmung von CPS 1 im Rahmen einer Kombinationsmessung liegt daher ausdrücklich ebenfalls im Rahmen der vorliegenden Erfindung.

Für eine solche Kombinationsmessung können alle dafür heute zur Verfügung stehenden modernen Messtechniken (z.B. Chiptechnologie) und Auswerttechniken (Computer mit entsprechenden Auswertalgorithmen) genutzt werden.

Aufgrund des frühzeitigen Auftretens der CSP 1 im Blut bietet sich die Durchführung einer CPS 1-Bestimmung auch im Rahmen der Prüfung von neuen Arzneimitteln während der Arzneimittelentwicklung an, um mögliche leberschädigende Wirkungen der untersuchten Arzneimittelkandidaten möglichst frühzeitig zu erkennen und spätere Gesundheitsschäden bei Patienten und finanzielle Einbußen und Imageschäden auf Herstellerseite zu vermeiden.

## Patentansprüche

1. *In vitro* Verfahren zur Früherkennung von arzneimittelinduzierten oder suchtmittelinduzierten Leberschäden und zur Erkennung der bereits erreichten Stufe der Leberschädigung, **dadurch gekennzeichnet, dass** man in Serum- oder Plasmaproben von Patienten, die mit potentiell leberschädigenden Arzneimitteln behandelt werden oder wurden und/oder die schädliche Genuss- und Suchtmittel zu sich nehmen und/oder die gegen potentiell lebertoxische Substanzen exponiert sind, das Auftreten des humanen Enzyms Carbamoylphosphat Synthase 1 (CPS 1) oder dessen Konzentration sowie außerdem das Auftreten einiger oder aller Leberenzyme bestimmt, die ausgewählt sind aus GOT (Glutamat-Oxalacetat-Transaminase), GPT (Serum-Glutamat-Pyruvat-Transaminase), GLDH (Glutamat-Dehydrogenase), γ-GT (γ-Glutamyl-Transpeptidase), Sorbitol-Dehydrogenase, alkalische Phosphatase, ALT (Alanin-Amino-Transferase), AST (Aspartat-Amino-Transferase), GDH (Glycerinphosphat-Dehydrogenase), LHD (Lactat-Dehydrogenase), Cholinesterase, LAP (Leucinamino-Peptidase), GGTP (Glutamyl-Transpeptidase), und aus der gleichzeitigen Nachweisbarkeit oder Nichtnachweisbarkeit der genannten Leberenzyme neben CPS 1 Schlüsse bezüglich der bereits erreichten Stufe der Leberschädigung ableitet.

2. Verfahren nach Anspruch 1, **dadurch gekennzeichnet, dass** man die Bestimmung von CPS 1 auf immundiagnostischem Wege durchführt.

3. Verfahren nach Anspruch 2, **dadurch gekennzeichnet, dass** man die immundiagnostische Bestimmung von CPS 1 mit Hilfe eines für humane CPS 1 selektiven Ligandenbindungsassays vom Sandwichtyp durchführt.

4. Verfahren nach einem der Ansprüche 1 bis 3, **dadurch gekennzeichnet, dass** man die Bestimmung von CPS 1 mit Hilfe von Bestimmungsverfahren durchführt, die das vollständige Enzym CPS 1 und/oder Fragmente davon erkennen.

5. Verfahren nach einem der Ansrüche 1 bis 4, **dadurch gekennzeichnet, dass** es als Schnelltest oder Point-of-Care-Test ausgestaltet ist.

6. Verfahren nach Anspruch 1, **dadurch gekennzeichnet, dass** man die Bestimmung von CPS 1 als Bestimmung der CPS 1-Enzymaktivität durchführt.

7. Verfahren nach einem der Ansprüche 1 bis 6, **dadurch gekennzeichnet, dass** man CPS 1 in Serum- oder Plasmaproben von Patienten bestimmt, die mit Arzneimitteln behandelt werden, die ausgewählt sind aus nicht-steroidalen antiinflammatorischen Arzneimitteln (NSAIDs), anderen Schmerzmitteln, anabolen Steroiden, hormonalen Kontrazeptiva, Gichtmitteln, Statinen, Inhalationsnarkotika, Antibiotika, Sulfonamiden, Tuberkulostatika, Antitumormitteln, Psychopharmaka, Herz-Kreislauf-Mitteln, Phytopharmaka, Paracetamol.

8. Verfahren nach Anspruch 7, **dadurch gekennzeichnet, dass** die Behandlung der Patienten eine Behandlung mit Arzneimitteln umfasst, die ausgewählt sind aus Tetracyclinen, Ibuprofen, Chlorpromazin, Captopril, Enalapril, Halothan, Kavakava, Streptomycin, Isoniazid und/oder Rifampicin.

## Claims

1. An *in vitro* method for the early detection of liver damages caused by drugs or addictive substances and for detecting the stage of liver damage already reached, **characterized in that** in serum or plasma samples of patients who are being treated or have been treated with potentially liver-damaging drugs and/or who take harmful stimulants and addictive substances and/or who are exposed against potentially hepatotoxic substances the occurrence of the human enzyme carbamoyl phosphate synthase (CPS 1) or its concentration are determined as well as the occurrence of some or all of the liver enzymes selected from GOT (glutamate oxalacetate transaminase), GPT (serum glutamate pyruvate transaminase), GLDH (glutamate dehydrogenase), gamma-GT (gamma-glutamyl transpeptidase), sorbitol dehydrogenase, alkaline phosphatase, ALT (alanine amino-transferase), AST (aspartate amino-transferase), GDH (glycerol phosphate dehydrogenase), LHD (lactate dehydrogenase), cholinesterase, LAP (leucine amino-peptidase), GGPT (glutamyl transpeptidase), and drawing conclusions concerning the stage of liver damage already reached on the basis of the concomitant detectability or non-detectability of said liver enzymes along with CPS 1.

2. The Method of Claim 1, **characterized in that** the determination of CPS 1 is carried out immunodiagnostically.

3. The Method of Claim 2, **characterized in that** the immunodiagnostic determination of CPS 1 is carried out by means of a ligand binding assay of the sandwich-type selective for human CPS 1.

4. The Method of any of Claims 1 to 3, **characterized in that** the determination of CPS 1 is carried out with the use of assay methods detecting the complete enzyme CPS 1 and/or fragments thereof.

5. The Method according to any of Claims 1 to 4, **characterized in that** it is provided as a quick test or a point-of-care test.

6. The Method of Claim 1, **characterized in that** the determination of CPS 1 is carried out as a determination of the CPS 1 enzyme activity.

7. The Method of any of Claims 1 to 6, **characterized in that** CPS 1 is determined in serum or plasma samples of patients who are being treated with drugs which are selected from nonsteroidal antiinflammatory drugs (NSAIDs), other analgesics, anabolic steroids, hormonal contraceptives, antigout agents, statins, inhalational anesthetics, antibiotics, sulfonamides, tuberculostatic agents, antitumor agents, psychotropic drugs, cardiovascular agents, phytopharmaceutical drugs, paracetamol.

8. The Method of Claim 7, characterized that the treatment of the patients comprises a treatment with drugs which are selected from tetracyclines, ibuprofen, chloropromazine, captopril, enalapril, halothane, kava kava, streptomycin, isoniazide and/or rifampicin.

## Revendications

1. Procédé in vitro pour le diagnostic précoce, pour la détection de dommages hépatiques induits par des médicaments ou induits par des drogues et pour la détection du degré déjà atteint par les dommages hépatiques, **caractérisé en ce que**, dans des échantillons de sérum ou de plasma de patients, qui sont traités ou ont été traités avec des médicaments potentiellement nocifs pour le foie et / ou qui consomment des produits de luxe ou des drogues et / ou qui sont exposés à des substances potentiellement toxiques au niveau hépatique, on détecte la présence de l'enzyme humain carbamoyl phosphate synthétase 1 (CPS 1) ou la concentration de celui-ci, ainsi que, en outre, la présence de quelques-uns ou de tous les enzymes hépatiques, qui sont choisis parmi les GOT (transaminase glutamo oxaloacétique), GPT (transaminase glutamo pyruvique sérique), GLDH (glutamate déshydrogénase), γ-GT (γ-glutamil transpeptidase), sorbitol déshydrogénase, phosphatase alcaline, ALT (aspartate amino transférase), GDH (glycorine phosphate déshydrogénase), LHD (lactate déshydrogénase), cholinestérase, LAP (leucine amino peptidase), GGTP (glytamil transpeptidase), et, du caractère détectable ou non détectable desdits enzymes hépatiques concurremment à CPS 1, on tire des conclusions au sujet du degré déjà atteint par les dommages hépatiques.

2. Procédé selon la revendication 1, **caractérisé en ce que** la détermination de CPS 1 est effectuée par voies immunodiagnostiques.

3. Procédé selon la revendication 1, **caractérisé en ce que** la détermination immunodiagnostique de CPS 1 est effectuée à l'aide d'un essai de liaison de ligand sélectif pour la CPS 1 humaine.

4. Procédé selon l'une des revendications 1 à 3, **caractérisé en ce que** la détermination immunodiagnostique de CPS 1 est effectuée à l'aide de procédés de détermination, qui détectent l'enzyme CPS 1 complet et /ou des fragments de celui-ci.

5. Procédé selon l'une des revendications 1 à 4, **caractérisé en ce que** celui-ci est conçu en tant que test rapide ou en tant que test point-of-care.

6. Procédé selon la revendication 1, **caractérisé en ce que** l'on effectue la détermination de CPS 1 en tant que détermination de l'activité enzymatique CPS 1.

7. Procédé selon l'une des revendications 1 à 6, **caractérisé en ce que** l'on détermine CPS 1 dans des échantillons de sérum ou de plasma de patients, qui sont traités avec des médicaments choisis dans le groupe comprenant des médicaments anti-inflammatoires, non stéroïdiens (NSAID), d'autres médicaments antalgiques, des stéroïdes anabolisants, des contraceptifs hormonaux, des médicaments contre la goutte, des statines, des narcotiques d'inhalation, des antibiotiques, des sulfonamides, des produits tuberculostatiques, tumoricides, psychotropes, cardiovasculaires, phytopharmaceutiques, le paracétamol.

8. Procédé selon la revendication 7, **caractérisé en ce que** le traitement des patients comprend un traitement avec des médicaments, qui sont choisis dans le groupe comprenant tétracyclines, ibuprofène, chlorpromazine, captopril, énalapril, halothane, kavakava, streptomycine, isoniazide et / ou rifampicine.
